(19) **Europäisches Patentamt / European Patent Office / Office européen des brevets**

(11) **EP 3 314 248 B1**

(12) **FASCICULE DE BREVET EUROPEEN**

(45) Date de publication et mention
de la délivrance du brevet:
**28.08.2019 Bulletin 2019/35**

(21) Numéro de dépôt: **16739212.5**

(22) Date de dépôt: **10.06.2016**

(51) Int Cl.:
***G01N 27/74*** *(2006.01)*

(86) Numéro de dépôt international:
**PCT/FR2016/051408**

(87) Numéro de publication internationale:
**WO 2016/207509 (29.12.2016 Gazette 2016/52)**

(54) **APPAREIL DE MESURE D'UNE QUANTITE DE MATERIAU SUPERPARAMAGNETIQUE**

VORRICHTUNG ZUR QUANTITATIVEN MESSUNG EINES SUPERPARAMAGNETISCHEN MATERIALS

APPARATUS FOR MEASURING A QUANTITY OF SUPERPARAMAGNETIC MATERIAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.06.2015 FR 1555927**

(43) Date de publication de la demande:
**02.05.2018 Bulletin 2018/18**

(73) Titulaire: **Atware**
**92350 Le Plessis Robinson (FR)**

(72) Inventeur: **AUGAIS, Thierry**
**91410 Saint Cyr sous Dourdan (FR)**

(74) Mandataire: **Cabinet Plasseraud**
**66, rue de la Chaussée d'Antin**
**75440 Paris Cedex 09 (FR)**

(56) Documents cités:
**US-A1- 2007 155 024    US-A1- 2009 164 161**
**US-A1- 2014 266 175**

• **KRAUSE ET AL: "Magnetic particle detection by frequency mixing for immunoassay applications", JOURNAL OF MAGNETISM AND MAGNETIC MATERIALS, ELSEVIER SCIENCE PUBLISHERS, AMSTERDAM, NL, vol. 311, no. 1, 15 mars 2007 (2007-03-15) , pages 436-444, XP022036705, ISSN: 0304-8853, DOI: 10.1016/J.JMMM.2006.10.1164**

**Description**

La présente invention concerne un appareil de mesure d'une quantité de matériau superparamagnétique, ainsi qu'un procédé de mesure qui utilise cet appareil.

Un matériau superparamagnétique est un matériau magnétique non-linéaire qui ne présente pas d'hystérèse lorsque l'excitation magnétique qui est appliquée à ce matériau est variée périodiquement.

De nombreuses applications nécessitent de mesurer des quantités d'un matériau superparamagnétique, notamment la réalisation de dosages immunologiques, appelés immunotests. Dans de tels immunotests, des molécules d'une substance immunologique telles que des anticorps ou des antigènes sont complexées avec des billes d'un matériau superparamagnétique, et la quantité de substance immunologique est déterminée à partir de mesures de la quantité de matériau superparamagnétique.

Par exemple, le document US 2007/0155024 décrit une méthode et un dispositif pour détecter sélectivement des particules ferromagnétiques ou superparamagnériques, tels que connus avant la présente invention.

Il existe alors un besoin important pour des appareils de mesure de quantités de matériau superparamagnétique, qui présentent les qualités suivantes :

- l'appareil peut être fabriqué à coût réduit ;

- l'appareil provoque une consommation énergétique qui est faible pour réaliser chaque mesure ;

- les résultats des mesures qui sont effectuées avec l'appareil ne sont pas affectés, ou sont peu affectés, par des décalages involontaires de signaux, appelés offsets ;

- les mesures qui sont effectuées avec l'appareil présentent un rapport signal-sur-bruit qui est élevé ;

- l'appareil produit des résultats de mesures qui sont robustes par rapport à des perturbations électromagnétiques extérieures ; et

- l'appareil doit satisfaire les exigences réglementaires de compatibilité électromagnétique (compatibilité CEM).

Pour améliorer ces critères par rapport aux appareils existants, un premier aspect de la présente invention propose un nouvel appareil qui est destiné à mesurer une quantité de matériau superparamagnétique, et qui comprend :

- quatre bobines de fil électrique possédant des géométries et des propriétés électriques et électromagnétiques respectives qui sont identiques ou sensiblement identiques, et ces quatre bobines étant connectées électriquement en série de façon à former une chaîne avec deux bornes extrêmes de la chaîne, une borne centrale dans la chaîne, et deux bornes secondaires de la chaîne qui sont situées chacune entre la borne centrale et l'une des deux bornes extrêmes,

- des moyens d'injection d'un courant continu dans la chaîne des bobines, qui sont connectés aux deux bornes extrêmes de la chaîne des bobines, et des moyens d'ajustement d'une intensité du courant continu ;

- des moyens d'injection d'un premier courant alternatif ayant une première fréquence, qui sont connectés pour injecter le premier courant alternatif dans la chaîne des bobines par la borne centrale, et pour reprendre ce premier courant alternatif par les deux bornes extrêmes, de sorte que le premier courant alternatif s'écoule avec des premières intensités qui sont identiques ou sensiblement identiques par les deux bornes extrêmes sans s'écouler par les bornes secondaires ;

- des moyens d'injection d'un second courant alternatif ayant une seconde fréquence différente de la première fréquence, qui sont connectés pour injecter le second courant alternatif dans la chaîne des bobines par les deux bornes secondaires, et pour reprendre ce second courant alternatif par la borne centrale et les deux bornes extrêmes de sorte que le second courant alternatif s'écoule avec des secondes intensités qui sont identiques ou sensiblement identiques par les deux bornes secondaires, et de sorte que ce second courant alternatif s'écoule avec des troisièmes intensités qui sont identiques ou sensiblement identiques dans toutes les bobines mais en sens contraires entre deux bobines qui sont successives dans la chaîne ; et

- des moyens de détection d'au moins une composante de tension électrique qui existe entre les deux bornes secondaires de la chaîne des bobines, une fréquence de cette composante de tension électrique, dite fréquence de mélange, étant une combinaison linéaire de la première fréquence et de la seconde fréquence, avec des coefficients de combinaison linéaire qui sont fixes, entiers et non nuls.

L'appareil de l'invention est ainsi adapté pour que lorsque la quantité de matériau superparamagnétique est située dans l'une des bobines, dite bobine de mesure, les moyens de détection produisent en sortie un signal de mesure qui est proportionnel à cette quantité de matériau superparamagnétique.

Dans l'appareil de l'invention, la quantité du matériau superparamagnétique est déterminée d'après l'ampleur de sa capacité à produire une tension alternative à la fréquence de mélange dans certaines au moins des bobines, du fait de la non-linéarité du matériau superparamagnétique.

Un tel appareil peut être fabriqué en utilisant des composants électriques qui sont disponibles commercialement à faibles coûts, notamment parce qu'ils sont déjà produits en grandes séries pour d'autres applications.

La connexion des moyens d'injection du courant continu aux bobines, celles des moyens d'injection des premier et second courants alternatifs, ainsi que la connexion des moyens de détection, réduisent ou suppriment par leurs sens de connexion des décalages qui seraient présents dans le signal de mesure, si bien que ce signal de mesure est effectivement proportionnel à la quantité du matériau superparamagnétique.

Par ailleurs, les moyens d'injection des courants peuvent être sélectionnés ou conçus aisément pour garantir la compatibilité électromagnétique.

De préférence, la bobine dans laquelle est située la quantité à mesurer du matériau superparamagnétique, peut être l'une des deux bobines qui sont intermédiaires entre les bornes secondaires de la chaîne des bobines.

Pour réduire les perturbations des mesures que pourraient provoquer des rayonnements électromagnétiques parasites, les quatre bobines peuvent être avantageusement juxtaposées les unes aux autres sur toute une longueur des bobines. En outre, les deux bobines qui sont intermédiaires entre les deux bornes secondaires de la chaîne des bobines peuvent avoir des sens d'enroulement qui sont opposés entre elles. Préférablement, les deux autres bobines peuvent aussi avoir des sens d'enroulement qui sont opposés entre ces deux autres bobines. De cette façon, un rayonnement électromagnétique parasite provoque des tensions d'induction dans les bobines qui s'annulent deux à deux.

Par ailleurs, un quotient entre les première et seconde fréquences peut être préférablement supérieur à 10, pour que les moyens de détection de la composante de tension électrique à la fréquence de mélange isolent cette composante avec une efficacité supérieure.

Dans des réalisations préférées d'un appareil conforme à l'invention, les moyens d'injection du courant continu, les moyens d'injection du premier courant alternatif et les moyens d'injection du second courant alternatif peuvent comprendre ensemble quatre câbles de connexion qui sont dédiés respectivement à chacune des quatre bobines. Chacun des câbles comprend deux fils électriques qui sont connectés un-à-une aux deux bornes successives dans la chaîne des bobines qui connectent directement la bobine à laquelle ce câble est dédié. Ainsi, tout le courant continu, tout le premier courant alternatif et tout le second courant alternatif qui sont injectés dans les bobines sont transportés en aller et retour par les quatre câbles. Alors, des longueurs respectives des quatre câbles peuvent être ajustées pour que les troisièmes intensités du second courant alternatif soient identiques ou sensiblement identiques en valeur absolue dans toutes les bobines de la chaîne. En outre, le transfert d'énergie électrique aux bobines, à partir des moyens d'injection du courant alternatif qui possède la fréquence la plus élevée, peut être amélioré de cette façon. La consommation énergétique de l'appareil, pour une sensibilité de mesure identique, peut ainsi être diminuée.

Par ailleurs, lorsque la seconde fréquence est supérieure à la première fréquence, les moyens d'injection du second courant alternatif peuvent comprendre une source du second courant alternatif et un enroulement primaire d'un transformateur, avec une sortie de courant de la source qui est connectée à un point milieu de l'enroulement primaire du transformateur. Les deux bornes extrêmes de l'enroulement primaire sont alors connectées respectivement aux deux bornes secondaires de la chaîne des bobines, et un enroulement secondaire du transformateur appartient aux moyens de détection de la composante de tension électrique qui possède la fréquence de mélange. Dans une telle réalisation, la tension qui est transmise aux moyens de détection possède, par conception de l'appareil, des composantes aux première et seconde fréquences qui ont des amplitudes nulles. Pour cette raison, l'appareil de l'invention peut être dit auto-compensé par rapport à des contributions éventuelles des signaux d'excitation aux première et seconde fréquences dans le signal qui est détecté. La composante de la fréquence de mélange peut ainsi être isolée avec une efficacité et une pureté qui sont supérieures.

En particulier pour obtenir un rapport signal-sur-bruit qui est supérieur, les moyens de détection de la composante de tension électrique qui possède la fréquence de mélange, dans un appareil conforme à l'invention, peuvent comprendre :

- un premier démodulateur synchrone qui est couplé aux moyens d'injection du second courant alternatif, et qui est agencé pour décaler en fréquence la composante de tension électrique qui possède la fréquence de mélange, par suppression d'une contribution de la seconde fréquence dans cette fréquence de mélange ; et

- un second démodulateur synchrone qui est couplé aux moyens d'injection du premier courant alternatif, et qui est agencé pour décaler en fréquence la composante de tension électrique qui possède la fréquence de mélange, par suppression d'une contribution de la première fréquence dans cette fréquence de mélange.

Ces premier et second démodulateurs synchrones sont alors agencés en cascade pour produire en sortie un signal électrique continu qui est proportionnel à une amplitude de la composante de tension électrique qui possède la fréquence de mélange. Ce signal constitue le signal de mesure, après amplification éventuelle. En outre, lorsque la seconde fréquence est supérieure à la première fréquence, les moyens d'injection du second courant alternatif peuvent comprendre des moyens pour multiplier une intensité instantanée du second courant alternatif par une séquence pseudo-aléatoire de facteurs chacun égaux à +1 ou -1, de façon à créer une modulation du second courant alternatif qui est aussi supprimée par le premier démodulateur synchrone. Le rapport signal-sur-bruit de l'appareil peut encore être augmenté de cette façon.

Eventuellement, pour augmenter encore la sensibilité des moyens de détection, ceux-ci peuvent être adaptés pour détecter une association de deux composantes de la tension alternative qui existe entre les deux bornes secondaires de la chaîne des bobines, correspondant à deux fréquences de mélange distinctes. Toutefois, dans un tel cas, ces deux composantes ont des contributions qui sont opposées pour la plus basse entre la première et la seconde fréquence dans les combinaisons linéaires qui constituent les deux fréquences de mélange, et des contributions qui sont identiques pour la plus élevée entre la première et la seconde fréquence.

De façon générale lorsque la seconde fréquence est supérieure à la première fréquence, les moyens d'injection du premier courant alternatif peuvent être connectés à la borne centrale de la chaîne des bobines par un premier condensateur qui est adapté pour être conducteur de courant alternatif à la première fréquence et à la seconde fréquence. Simultanément, les moyens d'injection du second courant alternatif peuvent être connectés à chacune des deux bornes secondaires de la chaîne des bobines par un second condensateur respectif qui est adapté pour être conducteur de courant alternatif à la seconde fréquence mais pas à la première fréquence. Une telle réalisation de l'appareil contribue à assurer la répartition dans les quatre bobines qui est voulue pour le courant continu, le premier courant alternatif et le second courant alternatif.

Un second aspect de l'invention propose un procédé pour mesurer une quantité inconnue d'un matériau superparamagnétique, qui comprend les étapes suivantes :

/1/ en utilisant un appareil conforme au premier aspect de l'invention, et avec une valeur de l'intensité du courant continu, et des valeurs respectives d'intensités pour le premier courant alternatif et pour le second courant alternatif, obtenir le signal de mesure qui est produit en sortie par les moyens de détection lorsqu'une quantité de référence du matériau superparamagnétique est située dans la bobine de mesure ; et

/2/ en utilisant le même appareil, la même valeur de l'intensité du courant continu qu'à l'étape /1/, et les mêmes valeurs d'intensités qu'à l'étape /1/ pour le premier courant alternatif et pour le second courant alternatif, obtenir le signal de mesure qui est produit en sortie par les moyens de détection lorsque la quantité inconnue du matériau superparamagnétique est située dans la bobine de mesure à la place de la quantité de référence.

La séquence des étapes /1/ et /2/ est exécutée à plusieurs reprises en variant entre chaque exécution de cette séquence la valeur de l'intensité du courant continu. Toutefois, la valeur d'intensité du premier courant alternatif et celle du second courant alternatif ne sont pas modifiées entre les exécutions successives des étapes /1/ et /2/. Le procédé comprend ensuite les étapes suivantes :

/3/ former un premier vecteur avec des valeurs des signaux de mesure qui ont été produits successivement par les moyens de détection lors des exécutions de l'étape /1/ ;

/4/ former un second vecteur avec des valeurs des signaux de mesure qui ont été produits successivement par les moyens de détection lors des exécutions de l'étape /2/ ; et

/5/ calculer la quantité inconnue du matériau superparamagnétique en multipliant la quantité de référence par un produit scalaire du premier vecteur avec le second vecteur, divisé par un produit scalaire du premier vecteur avec lui-même.

De préférence, les valeurs de l'intensité du courant continu qui sont utilisées pour les exécutions successives de la séquence des étapes /1/ et /2/, peuvent posséder une moyenne qui est nulle ou sensiblement nulle. Ainsi, des décalages continus qui seraient causés dans les résultats de mesure par les moyens d'injection du courant continu, ou par les moyens d'injection des premier et second courants alternatifs, sont réduits ou supprimés par effet de moyenne.

En particulier, le procédé de l'invention peut être utilisé pour des immunotests ou des dosages immunologiques. Pour de telles applications de l'invention, le matériau superparamagnétique peut être complexé avec une substance biomédicale ou biologique, notamment avec une substance immunologique, et en particulier avec un anticorps ou un antigène.

D'autres particularités et avantages de la présente invention apparaîtront dans la description ci-après d'exemples de réalisation non limitatifs, en référence aux dessins annexés, dans lesquels :

- la figure 1 est une vue en perspective de quatre bobines d'un appareil conforme à l'invention ;

- la figure 2 est un schéma synoptique d'un appareil conforme à l'invention ;

- la figure 3 correspond à la figure 2 pour des réalisations particulières des moyens d'injection de courants ; et

- la figure 4 est un diagramme synoptique d'un procédé de mesure d'une quantité de matériau superparamagnétique, en utilisant un appareil conforme à l'invention.

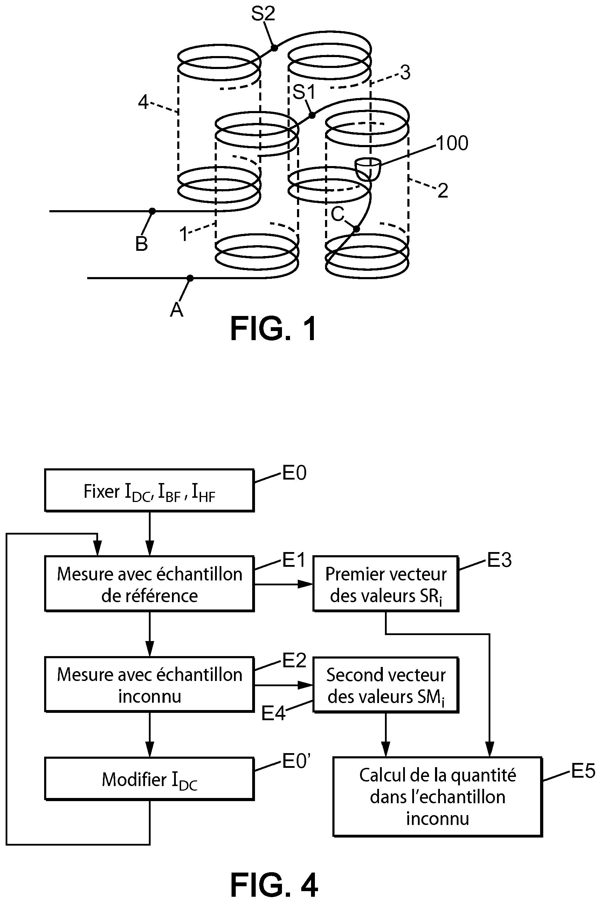

Pour raison de clarté de la figure 1, les dimensions apparentes des éléments qui y sont représentés ne correspondent ni à des dimensions réelles ni à des rapports de dimensions réels. En outre, des références identiques qui sont indiquées dans les figures 1 à 3 désignent des éléments identiques ou qui ont des fonctions identiques.

Dans la figure 1, les références 1 à 4 désignent quatre bobines de fil électrique qui sont conçues pour être identiques. Elles peuvent être disposées spatialement de façon quelconque les unes par rapport aux autres, mais la disposition de ces bobines côte-à-côte et parallèles les unes aux autres permet de compenser entre les bobines des tensions électriques parasites qui sont induites par des champs électromagnétiques externes. Les bobines 1 à 4 sont connectées électriquement en série, avec des sens d'enroulement du fil électrique dans chaque bobine qui peuvent être avantageusement inversés entre deux bobines successives dans la connexion en série. A et B désignent respectivement les bornes extrêmes de la connexion en série des bobines 1-4, la borne A étant du côté de la bobine 1 et la borne B du côté de la bobine 4. S1 et S2, dites bornes secondaires, sont respectivement intermédiaires entre les bobines 1 et 2, et entre les bobines 3 et 4, et C, dite borne centrale, est intermédiaire entre les bobines 2 et 3. Un logement est aménagé dans l'une des bobines, par exemple dans la bobine 2, pour insérer un échantillon 100 de matériau superparamagnétique à un emplacement qui est fixe dans la bobine. Cette bobine dans laquelle est placé l'échantillon a été appelée bobine de mesure dans la partie générale de la description.

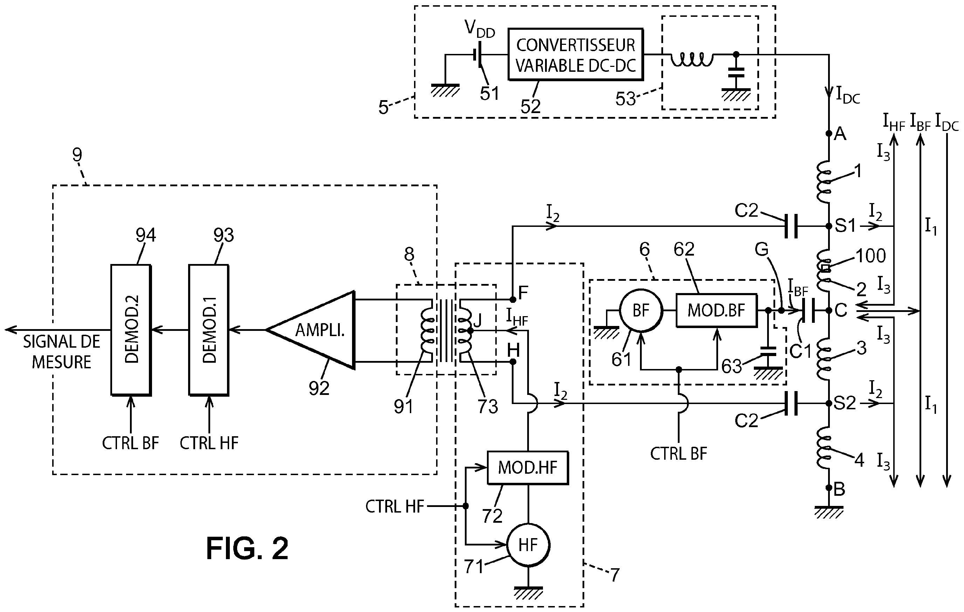

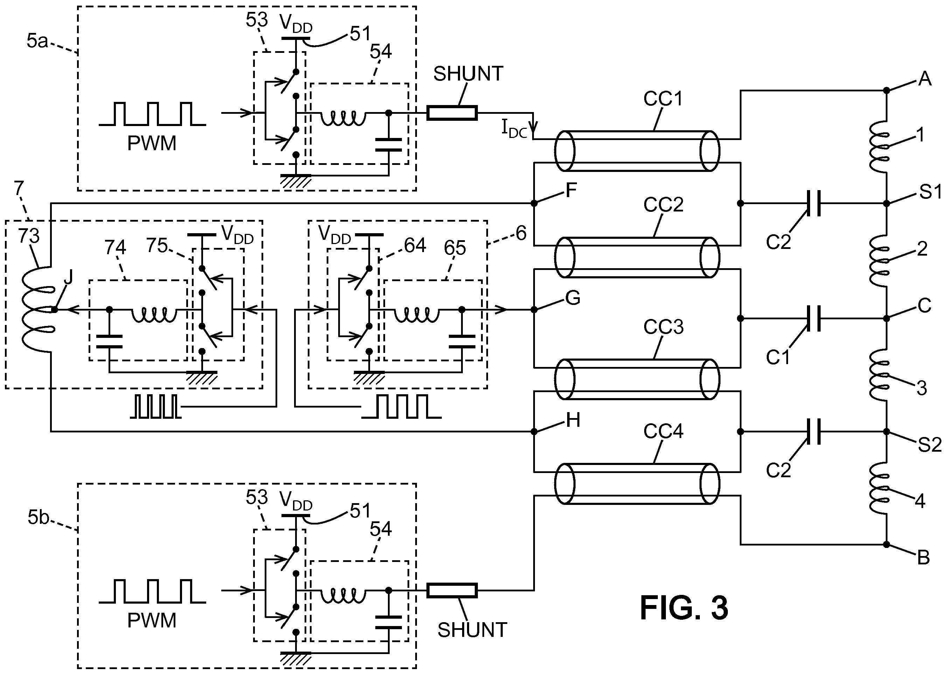

Les désignations qui ont été utilisées dans la partie générale de la présente description ont les correspondances suivantes avec celles du mode de réalisation des figures 2 et 3 : la première fréquence peut être égale à 1 kHz par exemple et est notée BF, et la seconde fréquence peut être égale à 200 kHz et est notée HF. Ces valeurs pour les fréquences BF et HF permettent d'utiliser des filtres qui sont disponibles commercialement, notamment pour des applications radio. Le premier et le second courants alternatifs, qui possèdent respectivement les fréquences BF et HF, sont notés $I_{BF}$ et $I_{HF}$. Le courant continu est noté $I_{DC}$.

Les références suivantes qui sont indiquées dans la figure 2 ont les significations données ci-dessous :

5    générateur du courant continu $I_{DC}$, appelé moyens d'injection du courant continu ;

6    générateur du courant $I_{BF}$, appelé moyens d'injection du premier courant alternatif ;

7    générateur du courant $I_{HF}$, appelé moyens d'injection du second courant alternatif ;

8    transformateur, avec un enroulement primaire référencé 73 et un enroulement secondaire référencé 91 ;

9    moyens de détection d'une composante de tension alternative qui existe entre les bornes secondaires S1 et S2, avec une fréquence de mélange qui est une combinaison linéaire des fréquences BF et HF ;

C1    condensateur dimensionné pour transmettre le courant $I_{BF}$, et qui transmet par conséquent aussi un courant à la fréquence HF ; et

C2    condensateurs dimensionnés pour transmettre des courants à la fréquence HF, mais pour empêcher des passages de courants à la fréquence BF.

Le générateur 5 peut être formé d'une source de tension continue 51, d'un convertisseur variable DC-DC 52 et d'un filtre 53. $V_{DD}$ est la tension continue qui est produite par la source 51. Le convertisseur 52 peut être commandé par un opérateur pour ajuster la valeur de l'intensité du courant $I_{DC}$ à une valeur voulue, puis pour varier cette valeur afin de réaliser une série de mesures successives. Le filtre 53 assure que des courants aux fréquences BF et HF puissent s'écouler hors des bobines 1-4 par la borne extrême A. Le générateur 5 produit le courant $I_{DC}$.

Le générateur 6 peut être formé d'une source de courant alternatif à la fréquence BF, référencée 61, et optionnellement d'un modulateur 62, noté MOD. BF. Lorsqu'il est utilisé, le modulateur 62 est connecté en série à la sortie de la source 61. CTRL BF désigne un signal de commande ou un signal de source du générateur 6. La sortie de la source 61, ou du modulateur 62, est connectée à la borne centrale C par l'intermédiaire du condensateur C1. Le condensateur 63 assure qu'un courant à la fréquence HF puisse s'écouler hors des bobines 1-4 par la borne C. Le générateur 6 produit le courant $I_{BF}$.

Le générateur 7 peut être formé d'une source de courant alternatif à la fréquence HF, référencée 71, optionnellement d'un modulateur 72, noté MOD. HF, et de l'enroulement primaire 73 du transformateur 8. Lorsqu'il est utilisé, le modulateur 72 est connecté en série à la sortie de la source 71. CTRL HF désigne un signal de commande ou un signal de source du générateur 7. La sortie de la source 71, ou du modulateur 72, est connectée à un point milieu J de l'enroulement primaire 73 du transformateur 8. Les bornes extrêmes F et H de l'enroulement primaire 73 sont connectées aux bornes secondaires S1 et S2 de la chaînes des bobines 1-4, respectivement, par l'intermédiaire des condensateurs C2. Le générateur 7 produit le courant $I_{HF}$.

Grâce à ces branchements, les quatre bobines 1-4 sont chacune parcourues par le courant continu $I_{DC}$, par une partie $I_1$ de l'intensité du courant $I_{BF}$ et aussi par une partie $I_3$ de l'intensité du courant $I_{HF}$, avec les orientations de courants qui sont indiquées dans la partie droite de la figure 2. En outre, les parties d'intensités $I_1$ et $I_3$ des deux courants alternatifs sont : $I_1 = I_{BF}/2$ et $I_3 = I_{HF}/4$. De plus, le courant $I_{HF}$ est injecté dans la chaîne des bobines 1-4 avec une intensité $I_2 = I_{HF}/2$ par chacune des bornes secondaires S1 et S2.

Les moyens de détection 9 comprennent l'enroulement secondaire 91 du transformateur 8, un amplificateur 92, noté AMPLI., un premier démodulateur synchrone 93, noté DEMOD. 1, et un second démodulateur synchrone 94, noté DEMOD. 2.

Le démodulateur synchrone 93 reçoit en entrée la tension qui existe aux bornes de l'enroulement secondaire 91, après amplification de cette tension, et reçoit simultanément le signal CTRL HF qui est introduit dans le générateur 7. Il est adapté pour effectuer un transfert de fréquence sur la composante de la tension dans l'enroulement secondaire 91 qui possède la fréquence HF-BF, et avantageusement aussi sur la composante de la même tension qui possède la fréquence HF+BF. De préférence, le démodulateur synchrone 93 peut être de type analogique. Lorsque le modulateur 72 est utilisé, la modulation du courant $I_{HF}$ qui est produite par ce modulateur 72 est automatiquement prise en compte par le démodulateur synchrone 93. Par exemple, cette modulation du courant $I_{HF}$ peut consister à inverser le sens du courant $I_{HF}$ à multiples reprises, selon une séquence temporelle aléatoire ou pseudo-aléatoire. De telles inversions de courant reviennent à multiplier le courant $I_{HF}$ par des facteurs successifs chacun égaux à +1 ou -1, pendant des durées variables, et produisent un étalement spectral du courant $I_{HF}$ tel que ce courant est délivré par le générateur 7.

Avantageusement, le signal qui est produit en sortie par le démodulateur synchrone 93 peut être amplifié avant d'être introduit dans le démodulateur synchrone 94.

Le démodulateur synchrone 94 reçoit donc en entrée le signal qui est produit par le démodulateur synchrone 93, après amplification éventuelle, et reçoit simultanément le signal CTRL BF qui est introduit dans le générateur 6. Il est adapté pour effectuer un transfert de fréquence sur le signal qui est produit par le démodulateur synchrone 93. De préférence, le démodulateur synchrone 94 peut être de type numérique. Lorsque le modulateur 62 est utilisé, la modulation du courant $I_{BF}$ qui est produite par ce modulateur 62 est automatiquement prise en compte par le démodulateur synchrone 94.

De cette façon, les moyens de détection 9 produisent en sortie une tension continue qui est proportionnelle à l'amplitude de la composante à la fréquence HF-BF, contenue dans la tension qui existe entre les bornes secondaire S1 et S2. Cette composante à la fréquence HF-BF résulte du comportement non-linéaire du matériau superparamagnétique de l'échantillon 100. Du fait de ce comportement non-linéaire, la réponse du matériau superparamagnétique aux excitations simultanées selon les deux fréquences HF et BF contient des signaux à des fréquences qui sont des combinaisons linéaires des deux fréquences HF et BF, avec des coefficients de combinaison linéaire qui sont entiers. Dans le cas présent, les démodulateurs synchrones 93 et 94 sélectionnent celle de ces combinaisons qui est la différence HF-BF. L'amplitude de la composante à cette fréquence HF-BF est alors proportionnelle à la quantité de matériau superparamagnétique qui est contenue dans l'échantillon 100. Cette amplitude constitue le signal de mesure, et peut être évaluée par un voltmètre.

La figure 3 illustre des modes de réalisation possibles pour les générateurs 5-7 qui sont particulièrement économiques, et une façon de les connecter aux bobines 1-4 qui permet d'améliorer le transfert d'énergie des générateurs 5-7 aux bobines 1-4.

Le générateur 5 de courant continu peut être formé par deux générateurs 5a et 5b qui sont identiques mais connectés respectivement aux bornes A et B de façon à pourvoir injecter le courant continu $I_{DC}$ dans la chaîne des bobines de la borne A vers la borne B ou en sens contraire. Un seul des deux générateurs 5a et 5b est donc activé pour chaque mesure, en fonction du signe qui est voulu pour le courant $I_{DC}$. Chaque générateur 5a, 5b comprend deux commutateurs qui sont connectés en série entre les deux bornes ($V_{DD}$ et masse) de la source 51. Un tel assemblage, désigné par la référence 54, est appelé couramment «bras de pont» dans le langage de l'Homme du métier. Il est destiné à une fonction de hachage. La borne intermédiaire entre les deux commutateurs de chaque générateur 5a, 5b est connectée à la sortie de ce générateur par l'intermédiaire du filtre 53. La commande des commutateurs par modulation de largeur d'impulsions, ou PWM pour «pulse width modulation» en anglais, permet d'ajuster la valeur absolue de l'intensité du courant continu $I_{DC}$. Des résistances, notées SHUNT peuvent être intercalées en série entre la sortie de chaque générateur 5a, 5b et la chaîne des bobines 1-4.

Le générateur 6 de courant alternatif peut aussi être réalisé à partir d'un bras de pont, référencé 64, et d'un filtre adapté 65. Le bras de pont du générateur 6 peut aussi être alimenté par la source 51, et ses deux commutateurs sont commandés alternativement à la fréquence BF. Le filtre 65, noté filtre BF, sélectionne la composante fondamentale pour transmission en sortie à la borne centrale C.

Enfin, le générateur 7 de courant alternatif peut encore être réalisé à partir d'un bras de pont, référencé 74, et d'un filtre adapté 75. Le bras de pont du générateur 7 peut être alimenté de même par la source 51, et ses deux commutateurs sont commandés alternativement à la fréquence HF. Le filtre 75 sélectionne un intervalle spectral autour de la composante fondamentale pour transmission en sortie aux bornes secondaires S1 et S2. De façon connue, une telle constitution du générateur 7 permet de réaliser simplement la modulation par étalement spectral, en décalant d'une demi-période les impulsions de commande du bras de pont selon une séquence temporelle aléatoire.

Les sorties des générateurs 5a, 5b et 6 ainsi que les deux bornes extrêmes F et H de l'enroulement primaire 73 du transformateur 8, peuvent être connectées aux bornes A, B, C et S1, S2 par quatre câbles bifilaires CC1-CC4. Les quatre câbles CC1-CC4 sont avantageusement d'un même type, bifilaire ou coaxial, de préférence blindé et à impédance caractéristique maîtrisée. Ils sont connectés de la façon suivante :

- le premier fil du câble CC1 relie la sortie du générateur 5a à la borne A de la chaîne des bobines 1-4 ;

- le second fil du câble CC1 et le premier fil du câble CC2 sont connectés en parallèle pour relier la borne extrême F de l'enroulement primaire du transformateur 8 à la borne secondaire S1 de la chaîne des bobines 1-4, par l'intermédiaire du premier condensateur C2 ;

- le second fil du câble CC2 et le premier fil du câble CC3 sont connectés en parallèle pour relier la sortie G du générateur 6 à la borne centrale C de la chaîne des bobines 1-4, par l'intermédiaire du condensateur C1 ;

- le second fil du câble CC3 et le premier fil du câble CC4 sont connectés en parallèle pour relier la borne extrême H de l'enroulement primaire du transformateur 8 à la borne secondaire S2 de la chaîne des bobines 1-4, par l'intermédiaire du second condensateur C2 ; et

- le second fil du câble CC4 relie la sortie du générateur 5b à la borne B de la chaîne des bobines 1-4.

Alors, les longueurs respectives des câbles CC1-CC4 peuvent être sélectionnées avantageusement pour ajuster les impédances de connexion qui sont effectives pour le courant alternatif $I_{HF}$. Ainsi, il est possible d'augmenter l'énergie électrique qui est transférée par le générateur 7 aux bobines 1-4, à consommation équivalente de l'appareil.

En référence à la figure 4, une première étape E0 d'un procédé de mesure d'une quantité de matériau superparamagnétique consiste à fixer une valeur initiale pour l'intensité du courant $I_{DC}$, et des valeurs pour les intensités des courants $I_{BF}$ et $I_{HF}$. Ces deux dernières valeurs, pour $I_{BF}$ et $I_{HF}$, sont utilisées sans modification pendant tout le procédé.

L'étape E1 consiste à effectuer une première mesure avec un échantillon de référence du matériau superparamagnétique. L'échantillon de référence est donc placé dans la bobine 2. La quantité de matériau superparamagnétique qui est contenue dans cet échantillon de référence est supposée connue, par exemple par une valeur de masse qui est notée $M_0$. La valeur du signal de mesure qui est obtenue pour cette première mesure est noté $SR_1$.

L'étape E2 est identique à l'étape E1, mais en remplaçant dans la bobine 2 l'échantillon de référence par l'échantillon à mesurer. Dans des utilisations simples de l'invention, l'échantillon à mesurer et l'échantillon de référence contiennent le même matériau superparamagnétique. Toutefois, la quantité qui est contenue dans l'échantillon à mesurer est inconnue, et le but du procédé est de la déterminer avec une fiabilité maximale. La valeur du signal de mesure qui est obtenue pour cette deuxième mesure est noté $SM_1$.

Lors de l'étape E0', la valeur de l'intensité du courant $I_{DC}$ est modifiée, puis les étapes E1 et E2 sont répétées avec cette nouvelle valeur pour $I_{DC}$. Des valeurs de signaux de mesures $SR_2$ et $SM_2$ sont alors obtenues pour l'échantillon de référence et pour l'échantillon à mesurer, respectivement.

Les étapes E0', E1 et E2 sont encore répétées, pour un total de N exécutions de la séquence des étapes E1 et E2, N étant un entier naturel supérieur à 1. Les valeurs de signaux de mesures $SR_i$ et $SM_i$ sont alors obtenues pour l'échantillon de référence et pour l'échantillon à mesurer, respectivement, à chaque exécution i de la séquence, i étant un indice entier de 1 à N. De préférence, les valeurs qui sont sélectionnées pour le courant $I_{DC}$ lors des exécutions des étapes E0 et E0' ont une valeur moyenne nulle ou sensiblement nulle. On entend par valeur moyenne sensiblement nulle une valeur moyenne qui est inférieure à la plupart des valeurs qui ont été sélectionnées pour le courant $I_{DC}$, en valeur absolue.

A l'étape E3, on construit un premier vecteur **SR** qui est constitué par les valeurs $SR_i$, relatives à l'échantillon de référence : **SR** = $[SR_i]$.

De la même façon, construit à l'étape E4 un second vecteur **SM** qui est constitué par les valeurs $SM_i$, relatives à l'échantillon à mesurer : **SM** = $[SM_i]$.

Alors, l'étape E5 consiste à calculer la masse M de matériau superparamagnétique qui est contenue dans l'échantillon à mesurer, à partir des deux vecteurs de valeurs qui ont été construits aux étapes E3 et E4. La masse M peut ainsi être calculée selon la formule :

$$M = M_0 \cdot (\textbf{SR} \times \textbf{SM})/(\textbf{SR} \times \textbf{SR}),$$

où x désigne le produit scalaire entre deux vecteurs.

Il est entendu que l'invention peut être reproduite en modifiant des aspects secondaires de celle-ci par rapport à la description détaillée qui a été donnée ci-dessus, tout en conservant certains au moins des avantages cités. Parmi ces modifications, on peut citer de façon non-limitative :

- les positions et orientations relatives des quatre bobines 1-4 peuvent être changées ;
- l'utilisation des modulateurs 62 et 72 peut être combinée avec la réalisation des générateurs 6 et 7 à partir de bras de ponts ;
- les générateurs 5, 6 et 7 peuvent avoir des constitutions différentes de celles décrites en relation avec les figures 2 et 3 ; et
- le mélange de fréquences qui est sélectionné par les moyens de détection 9 pour obtenir le signal de mesure, peut être une combinaison linéaire des fréquences BF et HF autre que la différence HF-BF et la somme HF+BF.

**Revendications**

1. Appareil de mesure d'une quantité de matériau magnétique non-linéaire sans hystérèse, dit matériau superparamagnétique, l'appareil comprenant :

   - quatre bobines (1-4) de fil électrique possédant des géométries et des propriétés électriques et électromagnétiques respectives qui sont identiques ou sensiblement identiques, et les quatre bobines étant connectées électriquement en série de façon à former une chaîne avec deux bornes extrêmes (A, B) de ladite chaîne, une borne centrale (C) dans ladite chaîne, et deux bornes secondaires (S1, S2) de ladite chaîne qui sont situées chacune entre la borne centrale et l'une des deux bornes extrêmes,
   - des moyens d'injection (5) d'un courant continu ($I_{DC}$) dans la chaîne des bobines (1-4), connectés aux deux bornes extrêmes (A, B) de la chaîne des bobines, et des moyens d'ajustement d'une intensité du courant continu ;
   - des moyens d'injection (6) d'un premier courant alternatif ($I_{BF}$) ayant une première fréquence (BF), connectés pour injecter ledit premier courant alternatif dans la chaîne des bobines (1-4) par la borne centrale (C), et pour reprendre ledit premier courant alternatif par les deux bornes extrêmes (A, B), de sorte que le premier courant alternatif s'écoule avec des premières intensités ($I_1$) qui sont identiques ou sensiblement identiques par lesdites deux bornes extrêmes sans s'écouler par les bornes secondaires (S1, S2) ;
   - des moyens d'injection (7) d'un second courant alternatif ($I_{HF}$) ayant une seconde fréquence (HF) qui est différente de la première fréquence (BF), connectés pour injecter ledit second courant alternatif dans la chaîne des bobines (1-4) par les deux bornes secondaires (S1, S2), et pour reprendre ledit second courant alternatif par la borne centrale (C) et les deux bornes extrêmes (A, B) de sorte que le second courant alternatif s'écoule avec des secondes intensités ($I_2$) qui sont identiques ou sensiblement identiques par lesdites deux bornes secondaires, et de sorte que ledit second courant alternatif s'écoule avec des troisièmes intensités ($I_3$) qui sont identiques ou sensiblement identiques dans toutes les bobines mais en sens contraires entre deux bobines qui sont successives dans la chaîne ; et
   - des moyens de détection (9) d'au moins une composante de tension électrique qui existe entre les deux bornes secondaires (S1, S2) de la chaîne des bobines (1-4), une fréquence de ladite composante de tension électrique, dite fréquence de mélange, étant une combinaison linéaire de la première fréquence (BF) et de la seconde fréquence (HF), avec des coefficients de combinaison linéaire fixes, entiers et non nuls,

   de sorte que lorsque la quantité de matériau superparamagnétique est située dans l'une (2) des bobines (1-4), dite bobine de mesure, les moyens de détection (9) produisent en sortie un signal de mesure qui est proportionnel à ladite quantité de matériau superparamagnétique.

2. Appareil selon la revendication 1, dans lequel les quatre bobines (1-4) sont juxtaposées les unes aux autres sur toute une longueur des bobines, et les deux bobines (2, 3) qui sont intermédiaires entre les deux bornes secondaires (S1, S2) de la chaîne des bobines ont des sens d'enroulement qui sont opposés entre lesdites deux bobines.

3. Appareil selon la revendication 1 ou 2, dans lequel un quotient entre les première (BF) et seconde (HF) fréquences est supérieur à 10.

4. Appareil selon l'une quelconque des revendications précédentes, dans lequel les moyens d'injection (5) du courant continu ($I_{DC}$), les moyens d'injection (6) du premier courant alternatif ($I_{BF}$) et les moyens d'injection (7) du second courant alternatif ($I_{HF}$) comprennent ensemble quatre câbles de connexion (CC1-CC4) qui sont dédiés respectivement à chacune des quatre bobines (1-4), chaque câble comprenant deux fils électriques connectés un-à-une aux deux bornes successives dans la chaîne des bobines qui connectent directement la bobine à laquelle ledit câble est dédié, de sorte que tout le courant continu, tout le premier courant alternatif et tout le second courant alternatif qui sont injectés dans les bobines soient transportés en aller et retour par lesdits câbles, et des longueurs respectives des dits câbles sont ajustées pour que les troisièmes ($I_3$) intensités du second courant alternatif ($I_{HF}$) soient identiques ou sensiblement identiques en valeur absolue dans toutes les bobines de la chaîne.

**5.** Appareil selon l'une quelconque des revendications précédentes, dans lequel la seconde fréquence (HF) est supérieure à la première fréquence (BF), et dans lequel les moyens d'injection (7) du second courant alternatif ($I_{HF}$) comprennent une source (71) du second courant alternatif et un enroulement primaire (73) d'un transformateur (8), une sortie de courant de ladite source étant connectée à un point milieu (J) de l'enroulement primaire du transformateur, et deux bornes extrêmes (F, H) dudit enroulement primaire étant connectées respectivement aux deux bornes secondaires (S1, S2) de la chaîne des bobines (1-4), et dans lequel un enroulement secondaire (91) du transformateur (8) appartient aux moyens de détection (9) de la composante de tension électrique qui possède la fréquence de mélange.

**6.** Appareil selon l'une quelconque des revendications précédentes, dans lequel les moyens de détection (9) de la composante de tension électrique qui possède la fréquence de mélange comprennent :

- un premier démodulateur synchrone (93) qui est couplé aux moyens d'injection (7) du second courant alternatif ($I_{HF}$), et qui est agencé pour décaler en fréquence la composante de tension électrique qui possède la fréquence de mélange, par suppression d'une contribution de la seconde fréquence (HF) dans ladite fréquence de mélange ; et
- un second démodulateur synchrone (94) qui est couplé aux moyens d'injection (6) du premier courant alternatif ($I_{BF}$), et qui est agencé pour décaler en fréquence la composante de tension électrique qui possède la fréquence de mélange, par suppression d'une contribution de la première fréquence (BF) dans ladite fréquence de mélange,

lesdits premier (93) et second (94) démodulateurs synchrones étant agencés en cascade de façon à produire en sortie un signal électrique continu qui est proportionnel à une amplitude de la composante de tension électrique qui possède la fréquence de mélange.

**7.** Appareil selon la revendication 6, dans lequel la seconde fréquence (HF) est supérieure à la première fréquence (BF), et les moyens d'injection (7) du second courant alternatif ($I_{HF}$) comprennent des moyens pour multiplier une intensité instantanée dudit second courant alternatif par une séquence pseudo-aléatoire de facteurs chacun égaux à +1 ou -1, de façon à créer une modulation dudit second courant alternatif qui est aussi supprimée par le premier démodulateur synchrone (93).

**8.** Appareil selon l'une quelconque des revendications précédentes, dans lequel la seconde fréquence (HF) est supérieure à la première fréquence (BF), et les moyens d'injection (6) du premier courant alternatif ($I_{BF}$) sont connectés à la borne centrale (C) de la chaîne des bobines (1-4) par un premier condensateur (C1) adapté pour être conducteur de courant alternatif à la première fréquence (BF) et à la seconde fréquence (HF) ; et les moyens d'injection (7) du second courant alternatif ($I_{HF}$) sont connectés à chacune des deux bornes secondaires (S1, S2) de la chaîne des bobines (1-4) par un second condensateur (C2) respectif adapté pour être conducteur de courant alternatif à la seconde fréquence (HF) mais pas à la première fréquence (BF).

**9.** Procédé de mesure d'une quantité inconnue d'un matériau superparamagnétique, comprenant les étapes suivantes :

/1/ en utilisant un appareil conforme à l'une quelconque des revendications précédentes, et avec une valeur de l'intensité du courant continu ($I_{DC}$), et des valeurs respectives d'intensités pour le premier courant alternatif ($I_{BF}$) et pour le second courant alternatif ($I_{HF}$), obtenir le signal de mesure qui est produit en sortie par les moyens de détection (9) lorsqu'une quantité de référence du matériau superparamagnétique est située dans la bobine de mesure (2) ; et

/2/ en utilisant le même appareil, et la même valeur de l'intensité du courant continu ($I_{DC}$) qu'à l'étape /1/, et les mêmes valeurs d'intensités qu'à ladite étape /1/ pour le premier courant alternatif ($I_{BF}$) et pour le second courant alternatif ($I_{HF}$), obtenir le signal de mesure qui est produit en sortie par les moyens de détection (9) lorsque la quantité inconnue du matériau superparamagnétique est située dans la bobine de mesure (2) à la place de la quantité de référence ;

exécuter la séquence des étapes /1/ et /2/ à plusieurs reprises en variant entre chaque exécution de ladite séquence la valeur de l'intensité du courant continu ($I_{DC}$), mais sans modifier les valeurs d'intensités du premier courant alternatif ($I_{BF}$) et du second courant alternatif ($I_{HF}$) ; puis :

/3/ former un premier vecteur avec des valeurs

($SR_i$) des signaux de mesure qui ont été produits successivement par les moyens de détection (9) lors des exécutions de l'étape /1/ ;

/4/ former un second vecteur avec des valeurs ($SM_i$) des signaux de mesure qui ont été produits successivement par les moyens de détection (9) lors des exécutions de l'étape /2/ ; et

/5/ calculer la quantité inconnue du matériau superparamagnétique en multipliant la quantité de référence par un produit scalaire du premier vecteur avec le second vecteur, divisé par un produit scalaire du premier vecteur avec lui-même.

10. Procédé selon la revendication 9, suivant lequel les valeurs de l'intensité du courant continu ($I_{DC}$) qui sont utilisées pour les exécutions successives de la séquence des étapes /1/ et /2/, possèdent une moyenne qui est nulle ou sensiblement nulle.

11. Procédé selon la revendication 9 ou 10, suivant lequel le matériau superparamagnétique est complexé avec une substance biomédicale ou biologique, notamment avec une substance immunologique, et en particulier avec un anticorps ou un antigène.


**Patentansprüche**

1. Vorrichtung zur Messung einer Quantität eines nichtlinearen magnetischen Materials ohne Hysterese, superparamagnetisches Material genannt, wobei die Vorrichtung aufweist:

   - vier Spulen (1-4) aus elektrischem Draht, die jeweilige Geometrien und elektrische und elektromagnetische Eigenschaften haben, die identisch oder im Wesentlichen identisch sind, wobei die vier Spulen elektrisch in Serie verbunden sind, um eine Kette mit zwei Endanschlüssen (A, B) der Kette, einem zentralen Anschluss (C) in der Kette und zwei Sekundäranschlüssen (S1, S2) der Kette, die sich jeweils zwischen dem zentralen Anschluss und einem der Endanschlüsse befinden, zu bilden,
   - Einrichtungen (5) zum Einspeisen eines Gleichstroms ($I_{DC}$) in die Kette der Spulen (1-4), die mit den zwei Endanschlüssen (A, B) der Kette der Spulen verbunden sind, und Einrichtungen zum Anpassen einer Intensität des Gleichstroms;
   - Einrichtungen (6) zum Einspeisen eines ersten Wechselstroms ($I_{BF}$) einer ersten Frequenz (BF), die verbunden sind, um den ersten Wechselstrom in die Kette der Spulen (1-4) über den zentralen Anschluss (C) einzuspeisen und um den ersten Wechselstrom über die zwei Endanschlüsse (A, B) wieder aufzunehmen, so dass der erste Wechselstrom mit ersten Intensitäten ($I_1$), die identisch oder im Wesentlichen identisch sind, über die zwei Endanschlüsse fließt, ohne über die zwei Sekundäranschlüsse (S1, S2) zu fließen;
   - Einrichtungen (7) zum Einspeisen eines zweiten Wechselstroms ($I_{HF}$) einer sich von der ersten Frequenz (BF) unterscheidenden zweiten Frequenz (HF), die verbunden sind, um den zweiten Wechselstrom in die Kette der Spulen (1-4) über die zwei Sekundäranschlüsse (S1, S2) einzuspeisen und um den zweiten Wechselstrom über den zentralen Anschluss (C) und die zwei Endanschlüsse (A, B) wieder aufzunehmen, so dass der zweite Wechselstrom mit zweiten Intensitäten ($I_2$), die identisch oder im Wesentlichen identisch sind, über die zwei Sekundäranschlüsse fließt, und so dass der zweite Wechselstrom mit dritten Intensitäten ($I_3$), die identisch oder im Wesentlichen identisch sind, in allen Spulen fließt, aber in entgegengesetzten Richtungen zwischen zwei Spulen, die in der Kette aufeinanderfolgend sind; und
   - Einrichtungen (9) zum Detektieren mindestens einer elektrischen Spannungskomponente, die zwischen den zwei Sekundäranschlüssen (S1, S2) der Kette der Spulen (1-4) vorhanden ist, wobei eine Frequenz der elektrischen Spannungskomponente, Mischfrequenz genannt, eine lineare Kombination der ersten Frequenz (BF) und der zweiten Frequenz (HF) ist, und wobei Koeffizienten der linearen Kombination fest, ganzzahlig und ungleich null sind,

so dass, wenn die Quantität des superparamagnetischen Materials sich in einer (2) der Spulen (1-4), Messspule genannt, befindet, die Detektionseinrichtungen (9) am Ausgang ein Messsignal hervorrufen, das proportional zu der Quantität des superparamagnetischen Materials ist.

2. Vorrichtung nach Anspruch 1, in welcher die vier Spulen (1-4) über eine ganze Länge der Spulen aneinander gereiht sind und die zwei Spulen (2, 3), die sich zwischen den zwei Sekundäranschlüssen (S1, S2) der Kette der Spulen befinden, untereinander entgegengesetzte Wicklungsrichtungen haben.

3. Vorrichtung nach Anspruch 1 oder 2, in welcher ein Quotient zwischen der ersten (BF) und zweiten (HF) Frequenz größer als 10 ist.

4. Vorrichtung nach einem der vorstehenden Ansprüche, in welcher die den Gleichstrom ($I_{DC}$) einspeisenden Einrichtungen (5), die den ersten Wechselstrom ($I_{BF}$) einspeisenden Einrichtungen (6) und die den zweiten Wechselstrom ($I_{HF}$) einspeisenden Einrichtungen (7) insgesamt vier Anschlusskabel (CC1-CC4) aufweisen, die jeweils einer der vier Spulen (1-4), zugeordnet sind, wobei jedes Kabel zwei elektrische Drähte aufweist, die eins-zu-eins mit zwei aufeinanderfolgenden Anschlüssen in der Kette der Spulen verbunden sind, die die Spule, der das Kabel zugeordnet ist, direkt verbinden, so dass der gesamte Gleichstrom, der gesamte erste Wechselstrom und der gesamte zweite Wechselstrom, die in die Spulen eingespeist werden, im Vor- und Rückgang von den Kabeln transportiert werden, und jeweilige Längen der Kabel angepasst sind, damit in allen Spulen der Kette die dritten Intensitäten ($I_3$) des zweiten Wechselstroms ($I_{HF}$) in ihrem Absolutwert identisch oder im Wesentlichen identisch sind.

5. Vorrichtung nach einem der vorstehenden Ansprüche, in welcher die zweite Frequenz (HF) höher als die erste Frequenz (BF) ist,
und in welcher die den zweiten Wechselstrom ($I_{HF}$) einspeisenden Einrichtungen (7) eine Quelle (71) des zweiten Wechselstroms und eine Primärwicklung (73) eines Transformators (8) aufweisen, wobei ein Stromausgang der Quelle mit einem Mittelpunkt (J) der Primärwicklung des Transformators verbunden ist und zwei Endanschlüsse (F, H) der Primärwicklung jeweils mit den zwei Sekundäranschlüssen (S1, S2) der Kette der Spulen (1-4) verbunden sind, und in welcher eine Sekundärwicklung (91) des Transformators (8) Teil der Detektionseinrichtungen (9) ist, die die elektrische Spannungskomponente, die die Mischfrequenz besitzt, detektieren.

6. Vorrichtung nach einem der vorstehenden Ansprüche, in welcher die Detektionseinrichtungen (9), die die elektrische Spannungskomponente, die die Mischfrequenz besitzt, detektieren, aufweisen:

 - einen ersten synchronen Demodulator (93), der mit den den zweiten Wechselstrom ($I_{HF}$) einspeisenden Einrichtungen (7) gekoppelt ist und der konfiguriert ist, eine Frequenzverschiebung an der elektrische Spannungskomponente, die die Mischfrequenz besitzt, durchzuführen durch Unterdrückung eines Beitrags der zweiten Frequenz (HF) in der Mischfrequenz,
 - einen zweiten synchronen Demodulator (94), der mit den den ersten Wechselstrom ($I_{BF}$) einspeisenden Einrichtungen (6) gekoppelt ist und der konfiguriert ist, eine Frequenzverschiebung an der elektrische Spannungskomponente, die die Mischfrequenz besitzt, durchzuführen durch Unterdrückung eines Beitrags der ersten Frequenz (BF) in der Mischfrequenz,

wobei der erste (93) und zweite (94) synchrone Demodulator kaskadenförmig angeordnet sind, um am Ausgang ein kontinuierliches elektrisches Signal zu erzeugen, das proportional zu einer Amplitude der elektrischen Spannungskomponente, die die Mischfrequenz besitzt, ist.

7. Vorrichtung nach Anspruch 6, in welcher die zweite Frequenz (HF) höher als die erste Frequenz (BF) ist, und die den zweiten Wechselstrom ($I_{HF}$) einspeisenden Einrichtungen (7) Einrichtungen aufweisen, um eine momentane Intensität des zweiten Wechselstroms mit einer pseudozufälligen Sequenz von Faktoren, jeder gleich +1 oder -1, zu multiplizieren, um eine Modulation des zweiten Wechselstroms zu erzeugen, die ebenfalls von dem ersten synchronen Demodulator (93) unterdrückt wird.

8. Vorrichtung nach einem der vorstehenden Ansprüche, in welcher die zweite Frequenz (HF) höher als die erste Frequenz (BF) ist,
und die den ersten Wechselstrom ($I_{BF}$) einspeisenden Einrichtungen (6) mit dem zentralen Anschluss (C) der Kette der Spulen (1-4) über einen ersten Kondensator (C1) verbunden sind, der angepasst ist, bei der ersten Frequenz (BF) und der zweiten Frequenz (HF) Wechselstromleiter zu sein;
und die den zweiten Wechselstrom ($I_{HF}$) einspeisenden Einrichtungen (7) mit jedem der Sekundäranschlüsse (S1, S2) der Kette der Spulen (1-4) über einen jeweiligen zweiten Kondensator (C2) verbunden sind, der angepasstf ist, bei der zweiten Frequenz (HF) Wechselstromleiter zu sein, aber nicht bei der ersten Frequenz (BF).

9. Verfahren zur Messung einer unbekannten Quantität eines superparamagnetischen Materials, aufweisend die folgenden Schritte:

 /1/ unter Verwendung einer Vorrichtung nach einem der vorstehenden Ansprüche und mit einem Wert der Intensität des Gleichstroms ($I_{DC}$) und jeweiligen Intensitätswerten für den ersten Wechselstrom ($I_{BF}$) und für den zweiten Wechselstrom ($I_{HF}$), Gewinnen des Messsignals, das von den Detektionseinrichtungen (9) am Ausgang hervorgerufen wird, wenn eine Referenzquantität des superparamagnetischen Materials in der Messspule (2) angeordnet ist; und
 /2/ unter Verwendung der gleichen Vorrichtung und des gleichen Werts der Intensität des Gleichstroms ($I_{DC}$) wie in Schritt /1/ und der gleichen Intensitätswerte wie in Schritt /1/ für den ersten Wechselstrom ($I_{BF}$) und für den zweiten Wechselstrom ($I_{HF}$), Gewinnen des Messsignals, das von den Detektionseinrichtungen (9) am Ausgang hervorgerufen wird, wenn anstelle der Referenzquantität die unbekannte Quantität des superparamagnetischen Materials in der Messspule (2) angeordnet ist;
Ausführen der Folge der Schritte /1/ und /2/ in mehreren Wiederaufnahmen, wobei zwischen jeder Ausführung der Folge der Intensitätswert des Gleichstroms ($I_{DC}$) geändert wird, aber die Intensitätswerte des ersten Wechselstroms ($I_{BF}$) und des zweiten Wechselstroms ($I_{HF}$) nicht geändert werden; dann

/3/ Bilden eines ersten Vektors mit Werten ($SR_i$) der Messsignale, die aufeinanderfolgend von den Detektionseinrichtungen (9) bei den Ausführungen des Schritts /1/ hervorgerufen worden sind;

/4/ Bilden eines zweiten Vektors mit Werten ($SM_i$) der Messsignale, die aufeinanderfolgend von den Detektionseinrichtungen (9) bei den Ausführungen des Schritts /2/ hervorgerufen worden sind; und

/5/ Berechnen der unbekannten Quantität des superparamagnetischen Materials durch Multiplizieren der Referenzquantität mit einem Skalarprodukt des ersten Vektors mit dem zweiten Vektor, dividiert durch ein Skalarprodukt des ersten Vektors mit sich selbst.

10. Verfahren nach Anspruch 9, gemäß welchem die Werte der Intensität des Gleichstroms ($I_{DC}$), die für die aufeinanderfolgenden Ausführungen der Folge der Schritte /1/ und /2/ verwendet werden, einen Mittelwert haben, der null oder im Wesentlichen null ist.

11. Verfahren nach Anspruch 9 oder 10, gemäß welchem das superparamagnetische Material mit einer biomedizinischen oder biologischen Substanz, insbesondere mit einer immunologischen Substanz und speziell mit einem Antikörper oder einem Antigen, komplexverbunden ist.


**Claims**

1. Device for measuring a quantity of a non-linear magnetic material devoid of hysteresis, known as a superparamagnetic material, the device comprising:

   - four coils (1-4) of electrical wire having identical or substantially identical respective geometries and electrical and electromagnetic properties, and the four coils being electrically connected in series so as to form a chain with two end terminals (A, B) of said chain, a central terminal (C) in said chain, and two secondary terminals (S1, S2) of said chain that are each located between the central terminal and one of the two end terminals,

   - means (5) for injecting a direct current ($I_{DC}$) into the chain of coils (1-4), connected to the two end terminals (A, B) of the chain of coils, and means for adjusting an intensity of the direct current;

   - means (6) for injecting a first alternating current ($I_{LF}$) having a first frequency (LF), connected to inject said first alternating current into the chain of coils (1-4) through the central terminal (C), and to recover said first alternating current through the two end terminals (A, B), so that the first alternating current flows with first intensities ($I_1$) that are identical or substantially identical through said two end terminals without flowing via the secondary terminals (S1, S2);

   - means (7) for injecting a second alternating current ($I_{HF}$) having a second frequency (HF) that is different from the first frequency (LF), connected to inject said second alternating current into the chain of coils (1-4) via the two secondary terminals (S1, S2), and to recover said second alternating current through the central terminal (C) and the two end terminals (A, B) so that the second alternating current flows with second intensities ($I_2$) that are identical or substantially identical via said two secondary terminals, and so that said second alternating current flows with third intensities ($I_3$) that are identical or substantially identical in all of the coils but in opposite directions between two successive coils in the chain; and

   - means (9) for detecting at least one voltage component that exists between the two secondary terminals (S1, S2) of the chain of coils (1-4), with a frequency of said voltage component, called mixing frequency, being a linear combination of the first frequency (LF) and the second frequency (HF), with linear combination coefficients that are fixed non-zero integers,

   so that when the quantity of superparamagnetic material is located in one (2) of the coils (1-4), known as the measuring coil, the detection means (9) output a measurement signal that is proportional to said quantity of superparamagnetic material.

2. Device according to claim 1, in which the four coils (1-4) are juxtaposed along an entire length of the coils, and the two coils (2, 3) that are in between the two secondary terminals (S1, S2) of the chain of coils have winding directions that are opposite to each other.

3. Device according to claim 1 or 2, in which a quotient between the first (LF) and second (HF) frequencies is greater than 10.

4. Device according any one of the preceding claims, in which the means (5) for injecting the direct current ($I_{DC}$), the means (6) for injecting the first alternating current ($I_{LF}$) and the means (7) for injecting the second alternating current ($I_{HF}$) together comprise four connection cables (CC1-CC4) that are respectively dedicated to each of the four coils (1-4), each cable comprising two electrical wires connected one-by-one to the two successive terminals in the chain of coils that directly connect the coil to which said cable is dedicated, so that all of the direct current, all of the first alternating current and all of the second alternating current that are injected into the coils are transported out and back by said cables, and respective lengths of said cables are adjusted so that the third intensities ($I_3$) of the second alternating current ($I_{HF}$) are identical or substantially identical in absolute terms in all the coils in the chain.

5. Device according to any one of the preceding claims, in which the second frequency (HF) is higher than the first frequency (LF), and in which the means (7) for injecting the second alternating current ($I_{HF}$) comprise a source (71) of the second alternating current and a primary winding (73) of a transformer (8), a current output of said source being connected to a middle point (J) of the primary winding of the transformer, and two end terminals (F, H) of said primary winding being connected respectively to the two secondary terminals (S1, S2) of the chain of coils (1-4), and in which a secondary winding (91) of the transformer (8) belongs to the means (9) for detecting the voltage component that has the mixing frequency.

6. Device according to any one of the preceding claims, in which the means (9) for detecting the voltage component that has the mixing frequency comprise:

    - a first synchronous demodulator (93) that is coupled to the means (7) for injecting the second alternating current ($I_{HF}$), and which is arranged in order to offset the frequency of the voltage component that has the mixing frequency, by suppressing a contribution of the second frequency (HF) to said mixing frequency; and
    - a second synchronous demodulator (94) that is coupled to the means (6) for injecting the first alternating current ($I_{LF}$), and which is arranged in order to offset the frequency of the voltage component that has the mixing frequency, by suppressing a contribution of the first frequency (LF) to said mixing frequency,

    said first (93) and second (94) synchronous demodulators being arranged in cascade so as to output a direct electric signal that is proportional to an amplitude of the voltage component that has the mixing frequency.

7. Device according to claim 6, in which the second frequency (HF) is higher than the first frequency (LF), and the means (7) for injecting the second alternating current ($I_{HF}$) comprise means of multiplying an instantaneous intensity of said second alternating current by a pseudo-random sequence of factors each equal to +1 or -1, so as to create a modulation of said second alternating current that is also suppressed by the first synchronous demodulator (93).

8. Device according to any one of the preceding claims, in which the second frequency (HF) is higher than the first frequency (LF), and the means (6) for injecting the first alternating current ($I_{LF}$) are connected to the central terminal (C) of the chain of coils (1-4) by a first capacitor (C1) suitable for conducting alternating current at the first frequency (LF) and at the second frequency (HF); and the means (7) for injecting the second alternating current ($I_{HF}$) are connected to each of the two secondary terminals (S1, S2) of the chain of coils (1-4) by a respective second capacitor (C2) suitable for conducting alternating current at the second frequency (HF) but not at the first frequency (LF).

9. Method for measuring an unknown quantity of a superparamagnetic material, comprising the following steps:

    /1/ using a device according to any one of the preceding claims, and with a value of the intensity of the direct current ($I_{DC}$), and respective intensity values for the first alternating current ($I_{LF}$) and for the second alternating current ($I_{HF}$), obtaining the measurement signal that is outputted by the detection means (9) when a reference quantity of the superparamagnetic material is located in the measuring coil (2); and
    /2/ using the same device, and the same value of the intensity of the direct current ($I_{DC}$) as in step /1/, and the same intensity values as in said step /1/ for the first alternating current ($I_{LF}$) and for the second alternating current ($I_{HF}$), obtaining the measurement signal that is outputted by the detection means (9) when the unknown quantity of the superparamagnetic material is located in the measuring coil (2) instead of the reference quantity;

    performing the sequence of steps /1/ and /2/ several times, varying the value of the intensity of the direct current ($I_{DC}$) between each performance of said sequence, but without modifying the intensity values of the first alternating current ($I_{LF}$) and the second alternating current ($I_{HF}$); then:

    /3/ forming a first vector with values ($SR_i$) of the measurement signals that have been outputted successively by the detection means (9) during the performances of step /1/;
    /4/ forming a second vector with values ($SM_i$) of the measurement signals that have been outputted successively by the detection means (9) during the performances of step /2/; and

/5/ calculating the unknown quantity of the superparamagnetic material by multiplying the reference quantity by a scalar product of the first vector and the second vector, divided by a scalar product of the first vector and itself.

10. Method according to claim 9, wherein the values of the intensity of the direct current ($I_{DC}$) that are used for the successive performances of the sequence of steps /1/ and /2/ have a mean value that is zero or substantially zero.

11. Method according to claim 9 or 10, wherein the superparamagnetic material is complexed with a biomedical or biological substance, in particular with an immunological substance, and particularly with an antibody or an antigen.

## FIG. 1

## FIG. 4

FIG. 2

**FIG. 3**

EP 3 314 248 B1

## RÉFÉRENCES CITÉES DANS LA DESCRIPTION

**Documents brevets cités dans la description**

- US 20070155024 A